(19) Europäisches Patentamt
European Patent Office
Office européen des brevets

(11) Publication number: **0 066 107**
**B1**

(12) # EUROPEAN PATENT SPECIFICATION

(45) Date of publication of patent specification: **14.08.85**

(21) Application number: **82103903.9**

(22) Date of filing: **05.05.82**

(51) Int. Cl.$^4$: **C 07 C 43/13,** A 61 K 7/00 // (C07C43/13, 41:03, 41:16, 41:26, C07D317:22, 303:22, 301:28, C07C31:125, 29:136)

(54) **2-Hydroxy-3-methyl-branched-alkoxypropyl glyceryl ether and cosmetic composition containing same.**

(30) Priority: **28.05.81 JP 81457/81**

(43) Date of publication of application:
**08.12.82 Bulletin 82/49**

(45) Publication of the grant of the patent:
**14.08.85 Bulletin 85/33**

(84) Designated Contracting States:
**AT CH DE FR GB LI**

(56) References cited:
**EP-A-0 024 806**
**EP-A-0 025 302**
**FR-A-1 575 466**
**FR-A-2 315 991**
**GB-A- 420 903**
**GB-A-1 343 502**
**GB-A-2 022 082**
**US-A-2 258 892**

**The file contains technical information submitted after the application was filed and not included in this specification**

(73) Proprietor: **KAO SOAP COMPANY, LTD.**
**1-14-1, Nihonbashi Kayaba-cho**
**Chuo-ku Tokyo (JP)**

(72) Inventor: **Takaishi, Naotake**
**1022-24, Himuro-cho Utsunomiya-shi**
**Tochigi-ken (JP)**
Inventor: **Inamoto, Yoshiaki**
**970-63, Himuro-cho Utsunomiya-shi**
**Tochigi-ken (JP)**
Inventor: **Urata, Kouichi**
**Ooaza Akabane 2606-6 Ichikai-machi**
**Haga-gun Tochigi-ken (JP)**
Inventor: **Kawano, Junichi**
**1-27-13, Nakashizu Sakura-shi**
**Chiba-ken (JP)**
Inventor: **Tsutsumi, Hisao**
**422-7, Ooaza Kokuno Miyashiro-machi**
**Minami-Saitama-gun Saitama-ken (JP)**

(74) Representative: **Wächtershäuser, Günter, Dr.**
**Tal 29**
**D-8000 München 2 (DE)**

**EP 0 066 107 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

1) *Field of the invention:*

This invention relates to a novel 2-hydroxy-3-methyl-branched-alkoxypropyl glyceryl ether (may hereinafter be abbreviated as "an α-monoalkyl ether of diglycerol") and a cosmetic composition containing same.

2) *Description of the prior art:*

In the natural world, there are a number of polyalcohol derivatives containing one or more ether bonds, among which monoalkyl ethers of glycerol (called "glyceryl ethers") are particularly well-known. For example, fish lipid contains palmityl glyceryl ether (chimyl alcohol), stearyl glyceryl ether (batyl alcohol) and oleyl glyceryl ether (serachyl alcohol).

The glyceryl ethers have found wide-spread commercial utility, particularly, as cosmetics bases and the like owing to their W/O-type emulsification characteristics (see, for example, Japanese Patent Laid-open Nos. 87612/1974, 92239/1974 and 12109/1977). Besides, it has been known that they have pharmacological effects such as erythropoietic stimulating effect for bone marrow, anti-inflammatory effect and antitumor activities (see, Japanese Patent Publication Nos. 10724/1974 and, especially, 18171/1977).

Taking into consideration that such glyceryl ethers are unique surfactants having many characteristic features, many attempts have been made to derive polyol ether compounds having a molecular structure analogeous to those of glyceryl ethers (in other words, containing one or more ether bonds and hydrophilic OH-groups therein) from polyalcohols (see, for example, U.S. Patent No. 2,258,892; Japanese Patent Publication No. 18170/1977 and Japanese Patent Laid-open Nos. 137905/1978 and 145224/1979). The thus-obtained polyol ether compounds are used as cosmetics bases (see, West German Patent Laid-open No. 2,455,287) and general emulsifiers owing to their W/O-type emulsification characteristics and also as antimicrobial and fungicidal agents.

Summary of the invention

Accordingly, an object of this invention is to provide a novel and useful α-monoalkyl ether of diglycerol.

Such an object has been achieved by an α-mono(methyl-branched alkyl) ether of diglycerol, which ether is represented by the general formula (I):

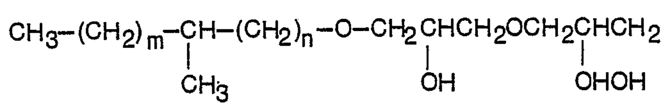

(I)

wherein *m* and *n* are integers and range respectively from 4 to 10 and from 5 to 11, $m + n$ ranges from 11 to 17, and the ether is distributed in its components with a peak at $m = 7$ and $n = 8$.

Detailed Description of Preferred Embodiments

The novel 2-hydroxy-3-methyl-branched-alkoxypropyl glyceryl ether of this invention, which is represented by the general formula (I), can be readily prepared with a high yield and purity from its corresponding glycidyl ether having the general formula (II):

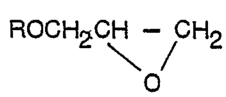

(II)

wherein R is 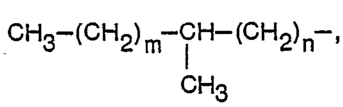

and *m* and *n* are as defined above, which glycidyl ether can in turn be easily prepared from its corresponding alcohol.

For instance, a desired α-mono(methyl-branched) alkyl ether of diglycerol may be obtained by adding in the presence of a basic or acidic catalyst a glycerol (III), whose hydroxyl groups at its 2-position and 3-position are protected by suitable protecting groups, to a glycidyl ether (II) to form a 1,3-dioxolane compound (IV) and then subjecting the 1,3-dioxolane compound (IV) to hydrolysis. This reaction is represented by the following formula:

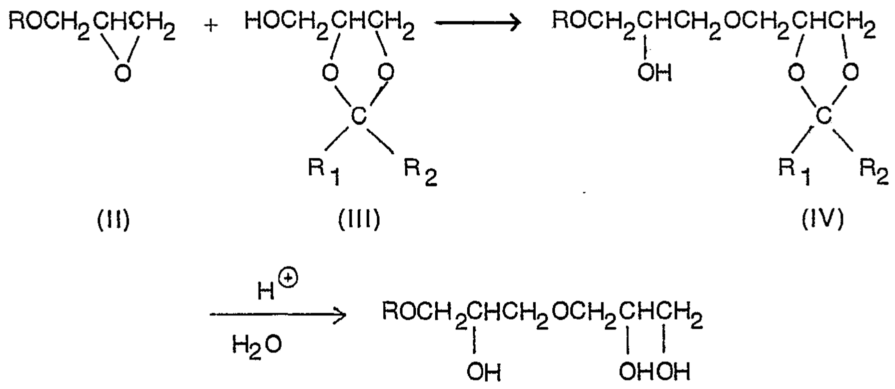


(II) (III) (IV)

(I)

wherein R has the same meaning as defined above, and $R_1$ and $R_2$ are individually a hydrogen atom or a lower alkyl, aryl or aralkyl group.

Although it is most preferred to produce the α-mono-(methyl-branched-alkyl) ether (I) of diglycerol according to this invention by the above-described process, it may also be prepared in accordance with any one of the following processes. However, where the following processes are employed, its purity is not sufficiently high and it is necessary in many instances to incorporate a purification step such as distillation step as a final step.

(1) Glycerol is reacted in the presence of an acidic or basic catalyst with a glycidyl ether (II);

(2) An alkali metal alcoholate of diglycerol is formed from diglycerol (VI) in the presence of an alkaline substance, to which alcoholate is reacted an alkyl halide or the like; and

(3) An alcohol (VII) is added in the presence of an acidic or basic catalyst to an epoxide compound (V) of the 1,3-dioxolane type and the resultant addition product (IV′) is then subjected to hydrolysis (Journal f. Parkt. Chemie, Band 316, 325—336 (1974)).

These processes may be represented by the following reaction formula:

(1) 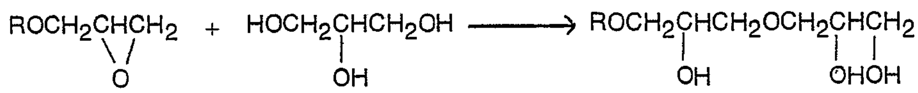

(II) (I)

(2) 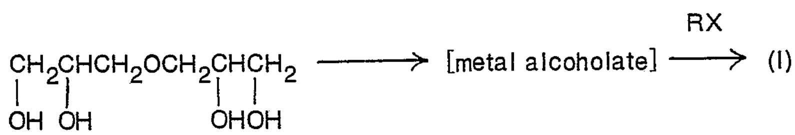


(VI)

(3) 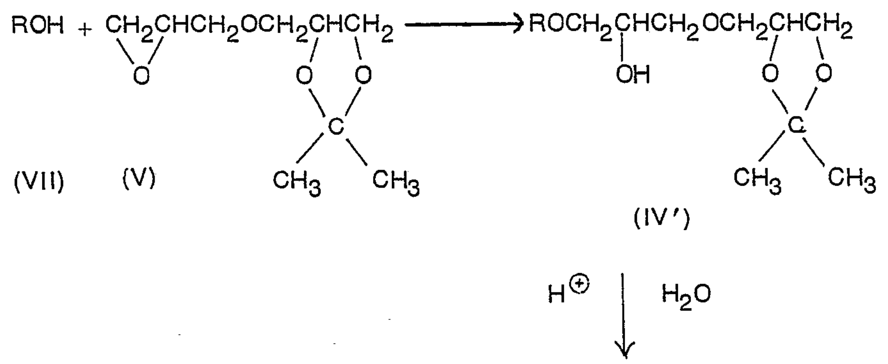


(VII) (V) (IV′)

(I)

wherein R is as defined above and X means a halogen atom.

New preparation processes have recently been developed to produce glycidyl ethers usable for preparing compounds of the present invention from their corresponding alcohols (ROH) with a high yield without need for isolating their corresponding halohydrin ethers (see, for example, Japanese Patent Laid-open Nos. 76508/1979, 141708/1979, 141709/1979 and 141710/1979). An alcohol is produced industrially as a mixture in which the total number of carbon atoms of its alkyl group and the position of its branched methyl group are distributed with its own characteristics. Accordingly, a glycidyl ether, which is derived from such an alcohol, is also obtained as a mixture having the same distribution. For example, isostearyl

alcohol which is a reduction product of isostearic acid having a methyl branch contains about 75% or more of isostearyl alcohol having 18 carbon atoms in total ($m + n = 15$) and the remainder of isostearyl alcohols respectively having 14, 16 and 20 carbon atoms in total. Its branched methyl group is located substantially at the centre of its main alkyl chain.—see, J. Amer. Oil. Chem. Soc. *51* 522 (1974).

As glycerols usable for preparing compounds of the present invention, i.e., glycerols in which hydroxyl groups at the 2-position and 3-position thereof are protected, there are acetals and ketals which are derived respectively from their corresponding aldehydes and ketones. As specific examples of compounds which are adapted to form protecting groups, namely, as aldehydes for obtaining acetals, may be mentioned for example aliphatic aldehydes (for example, formaldehyde, acetaldehyde, propionaldehyde, octylaldehyde, etc.), alicyclic aldehydes (for example, cyclopentylaldehyde, cyclohexylaldehyde, etc.) and aromatic aldehydes (benzaldehyde, naphthylaldehyde, etc.). On the other hand, as ketones for preparing ketals, may be mentioned for example aliphatic ketones (for example, acetone, methyl ethyl ketone, diethyl ketone, methyl propyl ketone, dipropyl ketone, ethyl propyl ketone, methyl hexyl ketone, etc.), alicyclic ketones (for example, cyclobutanone, cyclopentanone, cyclohexanone, cyclooctanone, etc.) and aromatic ketones (acetophenone, benzophenone, etc.). The above-described acetals and ketals, which are used in the preparation of compounds of this invention, may be prepared by subjecting glycerol and their corresponding aldehydes and ketones to dehydration and condensation reaction using an acidic substance as a catalyst.

As examples of the catalyst used when obtaining the 1,3-dioxolane compound (IV) may be mentioned, as basic catalysts, alkali metal hydroxides (for instance, LiOH, NaOH, KOH, etc.), alkali metal alcoholates (for example, NaOMe, NaOEt, t-BuOK, etc.) and tertiary amines (for instance, triethylamine, tributylamine, tetramethylethylene diamine, tetramethyl-1,3-diaminopropane, tetramethyl-1,6-diaminohexane, triethylene diamine, etc.). In addition, both protonic acids and Lewis acids may be used as acidic catalysts. Exemplary protonic acids include sulfuric acid, hydrochloric acid, nitric acid, phosphoric acid, etc., while, as Lewis acids, may be mentioned for example boron trifluoride ether complex, boron trifluoride-acetic acid complex, boron trifluoride-phenol complex, aluminum chloride, aluminum bromide, zinc chloride, tin tetrachloride, antimony chloride, titanium tetrachloride, silicon tetrachloride, ferric chloride, ferric bromide, cobaltic chloride, cobaltic bromide, zirconium chloride, boron oxide and acidic activated almina.

The 1,3-dioxolane compound (IV) may generally be synthesized by reacting one mole of an alkyl glycidyl ether (II) with 1—10 moles, or particularly preferably, 1—5 moles of an acetal or ketal (III) of glycerol, in the presence of 0.001—0.2 mole, or particularly preferably, 0.01—0.1 mole of a basic or acidic catalyst, at 70—150°C, or particularly preferably, 90—120°C.

The acetal or ketal of glycerol may theoretically be used in the equimolar amount as the glycidyl ether. Practically speaking, the reaction may proceed in a shorter period of time and a better yield may be available if the acetal or ketal of glycerol is used in an amount greater than the equimolar amount. Although the reaction proceeds without any reaction solvent, it is most preferable to use the acetal or ketal of glycerol in such an excess amount that it also serves as a solvent. A solvent may also be used if necessary. Any solvent may be used as a reaction solvent so long as it does not adversely affect on the reaction. However, it is suitable to use hydrocarbon solvents. Among such hydrocarbon solvents, there may be included aliphatic hydrocarbons such as pentane, hexane, heptane and octane, aromatic hydrocarbons such as benzene, toluene and xylene, alicyclic hydrocarbons such as cyclopentane and cyclohexane, and mixtures thereof.

When the reaction is carried out under the conditions mentioned above, the 1,3-dioxolane compound (IV) is usually obtained with a high yield of about 80% or higher. It may then be purified by virtue of distillation or the like. It may however be subjected to a hydrolysis reaction as is without need for conducting its isolation and purification, because it is generally obtained as a colorless, odor-free, clear liquid.

The hydrolysis reaction of the 1,3-dioxolane compound (IV) may be effected by any known method. It is however preferred to heat the 1,3-dioxolane compound (IV) in water, while using a protonic acid catalyst such as sulfuric acid, hydrochloric acid, nitric acid, phosphoric acid, benzenesulfonic acid or acetic acid. There is no special limitations vested to the amount of an acidic catalyst to be used. It is sufficient in quantity if used in an amount of 0.01—2N. Especially, it is suitable to use the acid catalyst in the range of 0.05—1.0 N. Water may be added with a water-soluble organic solvent, for example, a lower alcohol such as methanol, ethanol or isopropanol, THF, dioxane or the like. Te reaction is carrid out preferably at 50—100°C. Upon carrying out the hydrolysis reaction of the 1,3-dioxolane compound (IV) under such conditions, the intended product, the α-monoalkyl ether (I) of diglycerol (2-hydroxy-3-methyl-branched-alkoxypropyl glyceryl ether) can be obtained in a stoichiometric quantity.

The α-mono(methyl-branched-alkyl)ether of diglycerol according to this invention is chemically stable and excellent especially in emulsification capacity because it does not contain double bonds, ester bonds and the like in its molecule. Namely, when a compound according to this invention is used, a stable W/O-type emulsion can be formed even if lots of water is present (refer to Example 3).

Furthermore, when combined with a hydrophilic emulsifier having a broad HLB, an O/W-type emulsion can be resulted (refer to Example 4). Accordingly, it has been found that the α-mono(methyl-branched-alkyl) ether of diglycerol according to this invention is extremely useful as an emulsifier for emulsified cosmetic compositions.

Physical properties of a representative α-mono(methyl-branched-alkyl) ether according to this invention are as follows:

TABLE 1

| Methyl-branched alkyl group | m.p. | s.g. (25°C) | viscosity (30°) |
|---|---|---|---|
| Methyl-branched isostearyl ($m = 7$, $n = 8$; main component) | 5.5°C | 0.96 | 2040 cp |

TABLE 2

Solubility in Water

| 1) \ temp. | 25°C | 75°C |
|---|---|---|
| 10% | Dispersed liquid crystal | Undissolved (separated into two phases) |
| 50% | Stratified liquid crystal (opaque) | Stratified liquid crystal (clear) |
| 70% | Stratified liquid crystal (opaque) | Stratified liquid crystal (clear) |
| 90% | Hexagonal cylinder-like liquid crystal (opaque) | Dissolved intimately (clear) |

Note: 1) content of α-monomethyl-branched-isostearyl ether of diglycerol.

When an α-mono(methyl-branched-alkyl) ether of diglycerol is used as an emulsifier for a cosmetic composition, the cosmetic composition can be readily formulated into a cream or emulsion which features smoothness, good gloss and good stability as emulsion and excellent feeling upon its application owing to the above-described properties. It is most preferable to use the compound according to this invention as a component of emulsified cosmetic compositions.

Emulsified cosmetic compositions are used principally to protect the skin from dryness or environmental irritation, thereby avoiding the occurrence of roughened skin and maintaining the skin yound and fresh. As exemplary emulsified cosmetic compositions, may be mentioned vanishing cream, cold cream, emulsion, toilet water and the like.

The components of an emulsified cosmetic composition include water and oil besides the α-mono(methyl-branched-alkyl) ether of diglycerol according to this invention which is used as an emulsifier. As examples of such an oil, may be mentioned hydrocarbons such as liquid paraffin, vaseline, paraffin wax, squalene and ceresine wax; esters such as bees wax, spermaceti, carnauba wax, hydrous lanolin and synthetic esters of higher alcohols and fatty acids; vegetable oils such as olive oil, tsubaki oil, cotton seed oil and jojoba oil; alcohols such as long-chain, aliphatic alcohols and lanolin alcohol; and fatty acids such as stearic acid. An emulsified cosmetic composition may contain about 0.1—30% of an α-mono(methyl-branched-alkyl) ether of diglycerol, 1—98.9% of water and 1—60% of an oil, all by weight.

A part of the α-mono(methyl-branched-alkyl) ether of diglycerol as an emulsifier may be substituted by an emulsifier which has conventionally been employed as an emulsifier for cosmetic compositions. As examples of such a conventional emulsifier, there are polyoxyethylene alkyl ethers, polyoxyethylene sorbitan-fatty acid esters, polyoxyethylene-fatty acid esters, polyoxyethylene-hardened castor oil, polyoxyethylene sorbitol-fatty acid esters, cane sugar-fatty acid esters, sorbitan-fatty acid esters glycerol-fatty acid esters, fatty acid soap, alkyl sulfates, polyoxyethylenealkyl sulfates, alkyl phosphates and polyoxyethylenealkyl phosphates.

Furthermore, the emulsified cosmetic composition according to this invention may contain other components which have conventionally been employed to improve the quality of cosmetic compositions, if necessary. As such additional components, may be mentioned for example for moisturizers such as

glycerol, sorbitol, propylene glycol and 1,3-butylene glycol, perfumes; thickeners; pharmacologically effective agents; and preservatives.

The invention will hereinafter be described in further detail in the following examples. It shall however be noted that the present invention is not limited to such examples.

A preparation of an alcohol, which serves as a raw material for the glycidyl ether, will also be described as a referential example.

Referential Example 1

Into a 1 l, round bottomed flask equipped with a reflux condenser, thermometer, dropping funnel and stirrer, were added 120 g of a 50% aqueous solution of sodium hydroxide (60 g, i.e., 1.5 moles as pure sodium hydroxide), 68 g (0.25 mole) of monomethyl-branched isostearyl alcohol obtained in Referential Example 2, 200 ml of n-hexane and 2.51 g (0.0075 mole) of stearyl trimethylammonium chloride sequentially in the order as they appear. While maintaining the reaction mixture at a reaction temperature of 25°C in a water bath and agitating it at a stirring speed of 400 rpm, where dropped 93 grams (1 mole) of epichlorohydrin. After adding dropwise epichlorohydrin over about 1.5 hours, the reaction mixture was heated to 50°C and agitated at the same temperature for approximately further 8 hours. Upon completion of the reaction, the resultant reaction mixture was treated in accordance with procedures commonly employed, resulting in the provision of 68 g of monomethyl-branched isostearyl glycidyl ether represented by a formula given below (yield 83%).

Boiling point: 142—175°C (0.08 mmHg)

IR (liquid film, $cm^{-1}$: 3050, 3000, 1250, 1100, 920, 845.

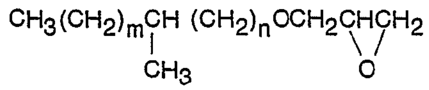

wherein *m* and *n* represented integers and range respectively from 4 to 10 and from 5 to 11, $m = n$ ranges from 11 to 17, and the ether is distributed in its components with a peak at $m + 7$ and $n = 8$.

Reference Example 2

In an 20l autoclave, were charged 4770 g of isopropyl isostearate ("Emery 2310", isopropyl isostearate, commercially available from Emery Corporation, U.S.A.) and 239 g of a copper -chromium catalyst (product of JGC Corporation). Then, the autoclave was filled with hydrogen gas to a pressure of 150 $kg/cm^2$. The reaction mixture was thereafter heated to 275°C. Hydrogen gas was blown into the autoclave for about 7 hours while maintaining the reaction system at 150 $kg/cm^2$ and 275°C. Upon cooling the reaction product and removing catalyst residue through filtration, 3500 g of a crude reaction product was obtained. By distilling the crude reaction product under reduced pressures, 3300 g of colorless, clear isostearyl alcohol was obtained as a fraction at 80—167°C/0.6 mmHg. The thus-obtained isostearyl alcohol (monomethyl-branched isostearyl alcohol) had an acid value of 0.05, saponification value of 5.5 and hydroxyl value of 181.4. Its IR (liquid film) spectrum contained absorptions respectively at 3340 and 1055 $cm^{-1}$, while absorptions occurred at δ3.50 (broad triplet, $—CH_2—OH$) in its NMR analysis ($CCl_4$ solvent). It was found from a gas chromatographic analysis of the above alcohol that its main component contained 18 carbon atoms as the total number of carbon atoms of its alkyl group and amounted to about 75% and the remainder was a mixture of those containing 14 and 16 carbon atoms as the total numbers of carbon atoms of their alkyl groups and having their pendant methyl groups near the centers of their main alkyl chains.

Example 1

(i) Into a 5 l, round bottomed flask equipped with a reflux condenser, thermometer, dropping funnel, nitrogen gas-feeding tube and stirrer, where charged 1061 g (8 moles) of glycerol dimethyl ketal and 28.4 g (0.165 mole) of tetramethyl-1,6-diaminohexane, which were then agitated and mixed while aerating the flask with nitrogen gas. Under the nitrogen gas aeration, 1308 g (4 moles) of the monomethyl-branched isostearyl glycidyl ether obtained in Referential Example 1 was dropped little by little through the dropping funnel. While the dropwise addition of the glycidyl ether, the reaction mixture was heated and maintained at 100°C or so. The glycidyl ether was added in the course of about 2 hours. In the course of the addition of the glycidyl ether, the temperature of the reaction mixture increased little by little and reached 125°C when the dropwise addition of the glycidyl ether was finished. The resultant reaction mixture was continuously heated and agitated for approximately further 6 hours at reaction temperatures of from 130 to 140°C. After verifying from a gas chromatographic diagram of the reaction mixture that the isostearyl glycidyl ether was completely used up, the reaction product was cooled down to room temperature. Then, 1500 g of city water and 100 g of salt were sequentially added to the reaction product. The resultant mixture was allowed to stand until it was separated into layers. The upper layer was collected and dried with anhydrous sodium sulfate, followed by the separation through distillation under reduced pressures of glycerol dimethyl ketal which was employed excessively. The resulting mixture was subjected to a further distillation under reduced pressures, thereby obtaining 2,2-dimethyl-4-(2'-hydroxy-3'-isostearoxy)propoxymethyl-1,3-dioxolane in an amount of 1510 grams (yield: 82%).

Boiling point: 210—230°C (0.5—0.8 mmHg).

Elemental analysis: Calculated for $C_{27}H_{54}O_5$:
C, 70.62; H, 11.85; O, 17.42.
Found: C, 70.7; H, 12.1; O, 16.9.

IR (liquid film, $cm^{-1}$):3460, 1380, 1370, 1260, 1210, 1115, 1055, 850.

NMR ($CCl_4$ solvent, $\delta$):

3.2 – 4.3 (multiplet, 12H;

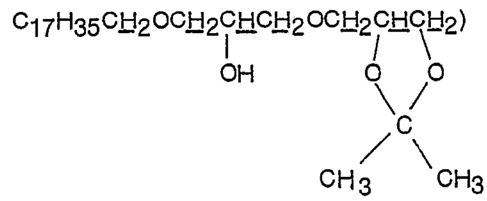

1.3 (singlet, 6H; –

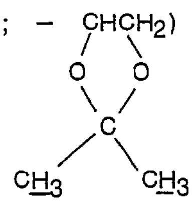

Acid value: 0.01 (calculated acid value: 0.0);
Saponification value: 1.5 (calculated saponification value: 0.0);
Hydroxyl value: 120 (calculated hydroxyl value: 122);
Iodine value: 1.0 (calculated iodine value: 0.0);
Oxirane oxygen: 0% (calculated oxirane oxygen: 0%); and
Molecular weight (determined by the VPO method in $CHCl_3$): 459 (calculated molecular weight: 459).

(ii) In a 2 l reaction vessel equipped with a stirrer, thermometer and reflux condenser, was charged 251 g (0.347 mole) of the 1,3-dioxolane compound obtained in the above Experiment (i), followed by a further addition of 230 ml of methanol and 300 ml of 1N-sulfuric acid. The mixture was heated and refluxed at 75—80°C while stirring the same. About 5 hours later, an gas chromatographic analysis showed that the hydrolysis of the 1,3-dioxolane compound was carried out completely. After allowing the reaction mixture to cool down to room temperature, it was allowed to stand and separate into an oil with water layers. The oil layer was collected. Subsequent to adding 500 ml of ether into the water layer, the resultant mixture was thoroughly shaken and then allowed to stand. The resultant ether layer was collected and combined with the oil layer which was previously obtained. The solvents were driven off under reduced pressures and the resultant substance was heated and dried for 3 hours at 100°C and 0.1 mmHg, leading to 220 g of colorless clear and syrupy 2-hydroxy-3-isostearoxypropyl glyceryl ether (yield: 96%).

Elemental analysis: Calculated for $C_{24}H_{50}O_5$:
C, 68.77; H, 12.02; O, 19.08.
Found: C, 68.4; H, 12.1; O, 19.2.

IR (liquid film, $cm^{-1}$):3360, 1105, 1040.

NMR ($CCl_4$ solvent, $\delta$):

3.2 – 3.8 (multiplet, 12H,

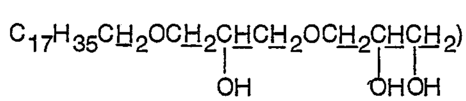

Acid value: 0.1 (theoretical value: 0.0);
Saponification value: 0.5 (theoretical value: 0.0);
Hydroxyl value: 400 (theoretical value: 402);
Iodine value: 0.5 (theoretical value: 0); and
Molecular weight (determined by the VPO method in $CHCl_3$): 420 (calculated molecular weight: 419).

Example 2

(i) The procedures of Experiment (i) in Example 1 were followed, except for the substitution of 24.3 g (0.165 mole) of 47% boron trifluoride-diethyl ether complex for tetramethyl-1,6-diaminohexane and the adoption of reaction temperatures of 85—90°C. The reaction product was neutralized and separated into a water and oil layers. The oil layer was collected and then subjected to distillation under reduced pressures, thereby obtaining 1450 g of 2,2-dimethyl-4-(2'-hydroxy-3'-methyl-branched-isostearoxy)propoxymethyl-1,3-dioxolane (yield: 79%). Its boiling point, and IR and NMR spectra were identical to those of 2,2-dimethyl-4-(2'-hydroxy-3'-isostearoxy)propoxymethyl-1,3-dioxolane obtained in Experiment (i) of Example 1.

Acid value: 0.02 (calculated acid value: 0.0);
Saponification value: 1.0 (calculated saponification value: 0.0);
Hydroxyl value: 125 (calculated hydroxyl value: 122);
Iodine value: 0.5 (calculated iodine value: 0.0); and
Oxirane oxygen: 0% (calculated value: 0%).

(ii) A hydrolysis reaction was carried out under the same conditions as those employed in Experiment (ii) of Example 1, whereby obtaining 225 g of colorless, clear, syrupy 2-hydroxy-3-isostearoxypropyl glyceryl ether (yield: 98%). Its IR and NMR spectra were identical to those obtained in Experiment (ii) of Example 1.

Acid value: 0.1 (calculated acid value: 0.0);
Saponification value: 0.3 (calculated saponification value: 0.0);
Hydroxyl value: 401 (calculated hydroxyl value: 402); and
Iodine value: 0.3 (calculated iodine value: 0.0).

Example 3

An emulsification test was conducted using the compound according to this invention, which was obtained in Example 1, and comparative compounds to compare their emulsification capacity. The emulsification test was carried out under the following conditions, using liquid paraffin as an oil. Test results are shown in Table 3.

(Emulsification test)

Ten parts by weight of liquid paraffin and 2 parts by weight of each sample compound were mixed together and heated to 70°C, followed by a gradual addition with stirring of 88 parts by weight of water (deionized water) which has also been heated at 70°C. After emulsification, the resultant mixture was cooled with stirring to room temperature, thereby obtaining an emulsion. This emulsion was put in test tubes. They were divided into two groups. One group of test tubes were stored at 40°C while the other group of test tubes were stored at 25°C. Their states were observed along the passage of days to compare their stability. By the way, the percentage oil phase separation and the percentage water phase separation are expressed respectively by the following equations:

$$\text{Percentage oil phase separation (\%)} = \frac{\text{Volume of oil phase separated (ml)}}{\text{12 (ml)}} \times 100$$

$$\text{Percentage water phase separation (\%)} = \frac{\text{Volume of water phase separated (ml)}}{\text{88 (ml)}} \times 100$$

TABLE 3

| | Compound tested | Emulsion type | State immediately after emulsification | State 3 days after emulsification (25°C) | |
|---|---|---|---|---|---|
| | | | | Percentage oil phase | Percentage water phase |
| 2) | Diglycerol α-mono-isostearyl ether (obtained in Example 1) | W/O | Uniform emulsion | 0 | 0 |
| Comparative compound | Glycerol monooleate | W/O | slightly uneven cream | 8 | 2 |
| | Glycerol monostearate | W/O | uneven cream | 100 | 100 |
| | Polyoxyethylene (5) oleyl ether | O/W | uniform emulsion | 0 | 5 |
| | Sorbitan monooleate | W/O | slightly uneven cream | 28 | 50 |
| | Sorbitan sesquioleate | W/O | slightly uneven cream | 14 | 48 |
| | α-monooleyl glyceryl ether | W/O | slightly uneven cream | 6 | 0 |
| | α-monoisostearyl glyceryl ether | W/O | uniform cream | 0 | 0 |

Note: 2) Product of this invention.

As readily envisaged from the above results, the compound according to this invention acted effectively as a W/O-type emulsifier. Thus, a stable W/O-type emulsion can be easily obtained when the above compound is used. However, when conventionally known emulsifiers were used, it was difficult to obtain a stable W/O-type emulsion. Even if a stable W/O-type emulsion was formed, its viscosity was high and it was impossible to obtain a W/O-type emulsion having a low viscosity.

Namely, it has been impossible according to the prior art technology to form a stable W/O-type emulsion having a low viscosity. Such an emulsion can however be easily obtained when a compound according to this invention is incorporated.

Example 4

Emulsified compositions were formulated incorporating the compound of this invention, which was prepared in Example 1, and comparative compounds. Their emulsion types, mean particle sizes and stability were investigated in accordance with the following methods. Investigation results are shown in Table 4.

(Emulsification test)

To 25 parts by weight of liquid paraffin, were added and mixed 4 parts by weight of a mixture of polyoxyethylene (POE) (20) sorbitan monooleate and each sample compound in its corresponding mixing ratio given in Table 4. The resultant mixture was heated to 70°C, to which 71 parts by weight of water (deionized water), which had been heated at 70°C, where slowly added with stirring to emulsify the mixture. After emulsification, it was cooled with stirring to room temperature, thereby obtaining an emulsion.

The thus-obtained emulsion was placed in a test tube and stored at 25°C. Its state was observed along the passage of days. Its stability was compared in accordance with the following evaluation standards.

| Evaluation | Definition |
|---|---|
| (−): | Uniform and no separation was visually observed. |
| (+): | Slightly separated oil phase or water phase was observed. |
| (++): | Distinctly separated oil or water phase was observed. |
| (+++): | Distinctly separated oil and water phases were both observed. |

The average particle size was determined by diluting each of the thus-prepared emulsions with an equeous 0.9% solution of sodium chloride and then measuring its particle sizes by a Coalter counter.

TABLE 4

| | Compound tested | Mixing ratio* | Emulsion type | Mean particle size | Stability of Emulsion | | |
|---|---|---|---|---|---|---|---|
| | | | | | 1 month at 40°C | 1 month at 25°C | 2 months at 25°C |
| Compound of this invention | Diglycerol α-monoisostearyl ether | 7/3 | O/W | 1.8 | – | – | – |
| | | 6/4 | O/W | 1.5 | – | – | – |
| | | 5/5 | O/W | 1.5 | – | – | – |
| | | 4/6 | O/W | 1.6 | – | – | – |
| | | 3/7 | O/W | 3.5 | – | – | – |
| Comparative compound | Sorbitan monooleate | 7/3 | O/W | 41.6 | +++ | ++ | +++ |
| | | 6/4 | O/W | 25.3 | +++ | ++ | +++ |
| | | 5/5 | O/W | 1.5 | – | – | – |
| | | 4/6 | O/W | 16.5 | ++ | – | + |
| | | 3/7 | O/W | 28.0 | +++ | ++ | +++ |
| | Glycerol monooleate | 7/3 | O/W | phase separation | +++ | +++ | +++ |
| | | 6/4 | O/W | phase separation | +++ | +++ | +++ |
| | | 5/5 | O/W | 1.5 | – | – | – |
| | | 4/6 | O/W | 1.8 | – | – | – |
| | | 3/7 | O/W | 30.5 | +++ | ++ | +++ |

Note: * $\frac{\text{Sample compound}}{\text{POE (20) sorbitan monooleate}}$ = Mixing ratio (by weight)

As apparent from Table 4, it is realized that a mixed emulsifier consisting in combination of the compound according to this invention and a hydrophilic emulsifier (POE (20) sorbitan monooleate in the above example) has a merit that its HLB width required to afford stable O/W-type emulsions, in other words, the range of their mixing ratio is broader than those of the conventionally known hydrophobic emulsifiers.

Accordingly, the compound according to this invention has such features that, when combined with a conventionally known hydrophilic emulsifier, it serves effectively as an O/W-type emulsifier too and it has a broad HLB suitable to yield stable O/W-type emulsions.

**Claims for the Contracting States: CH DE FR GB LI**

1. A 2-hydroxy-3-methyl-branched-alkoxypropyl glyceryl ether represented by the general formula (I):

$$CH_3-(CH_2)_m-\underset{CH_3}{\underset{|}{CH}}-(CH_2)_n-O-CH_2\underset{OH}{\underset{|}{CH}}CH_2OCH_2\underset{OH}{\underset{|}{CH}}\underset{OH}{CH_2} \qquad (I)$$

wherein *m* and *n* are integers and range respectively from 4 to 10 and from 5 to 11, $m + n$ ranges from 11 to 17, and the ether is distributed in its components with a peak at $m = 7$ and $n = 8$.

2. A cosmetic composition comprising a 2-hydroxy-3-methyl-branched-alkoxypropyl glyceryl ether represented by the general formula (I):

$$CH_3-(CH_2)_m-\underset{CH_3}{\underset{|}{CH}}-(CH_2)_n-O-CH_2\underset{OH}{\underset{|}{CH}}CH_2OCH_2\underset{OH}{\underset{|}{CH}}\underset{OH}{CH_2} \qquad (I)$$

wherein *m* and *n* are integers and range respectively from 4 to 10 and from 5 to 11, $m + n$ ranges from 11 to 17, and the ether is distributed in its components with a peak at $m = 7$ and $n = 8$.

3. The cosmetic composition according to Claim 2, wherein said cosmetic composition is of the emulsion type.

**Claims for the Contracting State: AT**

1. A cosmetic composition comprising a 2-hydroxy-3-methyl-branched-alkoxypropyl glyceryl ether represented by the general formula (I):

$$CH_3-(CH_2)_m-\underset{CH_3}{\underset{|}{CH}}-(CH_2)_n-O-CH_2\underset{OH}{\underset{|}{CH}}CH_2OCH_2\underset{OH}{\underset{|}{CH}}\underset{OH}{CH_2} \qquad (I)$$

wherein *m* and *n* are integers and range respectively from 4 to 10 and from 5 to 11, $m + n$ ranges from 11 to 17, and the ether is distributed in its components with a peak at $m = 7$ and $n = 8$.

2. The cosmetic composition according to Claim 1, wherein said cosmetic composition is of the emulsion type.

**Patentansprüche für die Vertragsstaaten: CH DE FR GB LI**

1. 2-Hydroxy-3-methyl-verzweigte Alkoxypropylglycerylether gemäß der allgemeinen Formel (I):

$$CH_3-(CH_2)_m-\underset{CH_3}{\underset{|}{CH}}-(CH_2)_n-O-CH_2\underset{OH}{\underset{|}{CH}}CH_2OCH_2\underset{OH}{\underset{|}{CH}}\underset{OH}{CH_2} \qquad (I)$$

wobei m und n ganze Zahlen sind und im Bereich von 4 bis 10 bzw. 5 bis 11 liegen, m + n im Bereich von 11 bis 17 liegt, und wobei die Verteilung der Komponenten des Ethers einen Spitzenwert bei m = 7 und n = 8 aufweist.

2. Kosmetisches Mittel, umfassend einen 2-Hydroxy-3-methylverzweigten Alkoxypropylglycerylether der allgemeinen Formel (I):

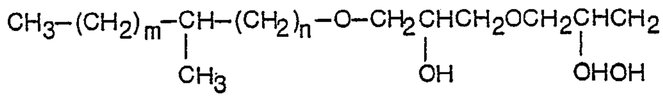

(I)

wobei m und n ganze Zahlen sind und im Bereich von 4 bis 10 bzw. 5 bis 11 liegen, m + n im Bereich von 11 bis 17 liegt, und wobei die Verteilung der Komponenten des Ethers einen Spitzenwert bei m = 7 und n = 8 aufweist.

3. Kosmetisches Mittel gemäß Anspruch 2, wobei das kosmetische Mittel vom Emulsions-Typ ist.

**Patentansprüche für den Vertragsstaat: AT**

1. Kosmetisches Mittel, umfassend einen 2-Hydroxy-3-methylverzweigten Alkoxypropylglycerylether der allgemeinen Formel (I):

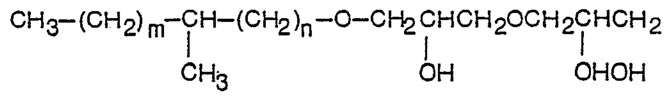

(I)

wobei m und n ganze Zahlen sind und im Bereich von 4 bis 10 bzw. 5 bis 11 liegen, m + n im Bereich von 11 bis 17 liegt, und wobei die Verteilung der Komponenten des Ethers einen Spitzenwert bei m = 7 und n = 8 aufweist.

2. Kosmetisches Mittel gemäß Anspruch 1, wobei das kosmetische Mittel vom Emulsions-Typ ist.

**Revendications pour les Etats contractants: CH DE FR GB LI**

1. Un 2-hydroxy-3-alcoxy à ramification méthyle propyl glycéryl éther représenté par la formule général (I):

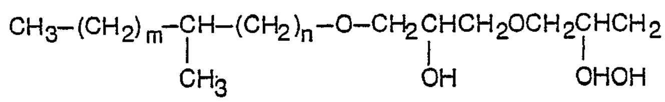

(I)

dans laquelle *m* et *n* sont des nombres entiers et se situent respectivement dans l'intervalle de 4 à 10 et de 5 à 11, *m* + *n* se situe dans l'intervalle de 11 à 17, et l'éther est distribué en ce qui concerne ses constituants avec un pic à *m* = 7 et *n* = 8.

2. Une composition cosmétique comprenant un 2-hydroxy-3-alcoxy à ramification méthyle propyl glycéryl éther représenté par la formule générale (I):

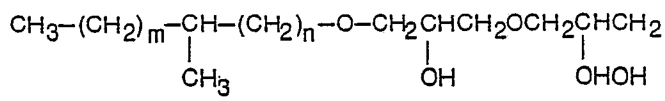

(I)

dans laquelle *m* et *n* sont des nombres entiers et se situent respectivement dans l'intervalle de 4 à 10 et de 5 à 11, *m* + *n* se situe dans l'intervalle de 11 à 17, et l'éther est distribué en ce qui concerne ses constituants avec un pic à *m* = 7 et *n* = 8.

3. La composition cosmétique selon la revendication 2, dans laquelle ladite composition cosmétique est du type émulsion.

**Revendications pour l'Etat contractant: AT**

1. Une composition cosmétique qui comprend un 2-hydroxy-3-alcoxy à ramification méthyl propyl glycéryl éther représenté par la formule générale (I)

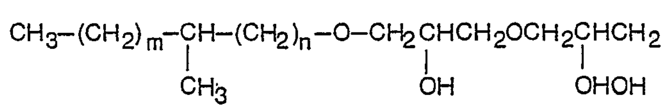

(I)

dans laquelle *m* et *n* sont des nombres entiers et se situent respectivement dans l'intervalle de 4 à 10 et de 5. à 11, *m* + *n* se situe dans l'intervalle de 11 à 17, et l'éther est distribué en ce qui concerne ses constituants avec un pic à *m* = 7 et *n* = 8.

2. La composition cosmétique selon la revendication 1, dans laquelle ladite composition cosmétique est du type émulsion.